Europäisches Patentamt

European Patent Office

Office européen des brevets

(19)

(11) **EP 1 264 578 A2**

(12) # DEMANDE DE BREVET EUROPEEN

(43) Date de publication:
**11.12.2002 Bulletin 2002/50**

(51) Int Cl.⁷: **A61B 5/103**

(21) Numéro de dépôt: **02291559.9**

(22) Date de dépôt: **19.05.1995**

(84) Etats contractants désignés:
**AT BE CH DE ES FR GB IT LI NL SE**

(30) Priorité: **20.05.1994 FR 9406187**

(62) Numéro(s) de document de la (des) demande(s) initiale(s) en application de l'article 76 CBE:
**95401167.2 / 0 682 912**

(71) Demandeur: **L'OREAL**
**75008 Paris (FR)**

(72) Inventeurs:
• **Guiolet, Alain**
**75019 Paris (FR)**

• **de Rigal, Jean**
**77410 Gressy (FR)**
• **Leveque, Jean-Luc**
**93340 Le Raincy (FR)**

(74) Mandataire: **Tanty, François**
**Nony & Associés,**
**3, rue de Penthièvre**
**75008 Paris (FR)**

Remarques:
Cette demande a été déposée le 21 - 06 - 2002 comme demande divisionnaire de la demande mentionnée sous le code INID 62.

(54) **Masque applicable sur la peau, apte à coopérer avec une tête d'application d'un appareil**

(57) L'invention se rapport à un masque applicable directement sur la peau, comportant un élément de masquage (18) muni d'un orifice (19) de forme asymétrique, et des supports adhésifs (22) auxquels l'élément de masquage est relié par des moyens de fixation amo-vibles (21).

FIG.3

EP 1 264 578 A2

**Description**

La présente invention concerne, selon un de ses aspects, un procédé pour la détermination de grandeurs caractéristiques du comportement d'une surface, notamment de la peau humaine, soumise à un rayonnement lumineux, et un appareil pour sa mise en oeuvre.

L'étude du comportement de la peau vis-à-vis d'un rayonnement lumineux présente un grand intérêt, en particulier pour la recherche de nouveaux produits cosmétiques, en particulier de protection solaire et pour la détermination de la sensibilité de différents types de peaux à l'exposition au soleil.

Concernant les produits solaires, on connaît l'échelle dite des indices de protection, qui est censée quantifier l'efficacité protectrice des différents produits solaires applicables sur la peau.

La méthode de détermination de ces indices de protection repose sur des déterminations cliniques qui ne peuvent présenter qu'une fiabilité relative.

Les méthodes in vivo utilisées à ce jour présentent elles aussi une fiabilité relative, principalement pour les deux raisons suivantes :

D'une part, le paramètre traditionnellement utilisé pour calculer l'indice de protection est une énergie lumineuse globale recueillie sur une peau éclairée sans distinguer les différents types d'énergies lumineuses qui peuvent provenir de cette surface.

D'autre part, les produits solaires les plus récents combinent des phénomènes à la fois chimiques et physiques d'absorption, de transformation et de réflexion de la lumière, tandis que les méthodes usuelles sont inopérantes devant la complexité de tels phénomènes.

Quant aux méthodes de détermination de la résistance d'une peau à l'exposition au soleil, elles consistent à irradier une petite surface de la peau, et à observer, dans un délai d'environ 24 heures, la réaction érythémale de la zone irradiée.

En procédant par essais successifs, on détermine le seuil à partir duquel le mécanisme d'auto-protection de la peau, qui se caractérise entre autres par le bronzage, devient insuffisant. La peau irradiée subit alors une brûlure qui se matérialise par un coup de soleil.

La présente invention vise, selon un de ses aspects, à fournir un procédé et un appareil permettant, avec une grande fiabilité, de caractériser le comportement d'une surface telle que la peau humaine vis-à-vis d'un rayonnement lumineux et, par suite, l'efficacité de protection des produits solaires applicables sur cette surface.

D'une manière générale, le procédé selon l'invention est caractérisé par les étapes suivantes :

- on éclaire une zone prédéterminée d'une surface avec un faisceau lumineux polarisé et orienté selon une direction d'incidence faisant, avec la normale à ladite surface, un angle compris entre 10° et 50°, et de préférence voisin de 35°,
- on récupère un signal lumineux de diffusion issu de la zone éclairée et orienté selon la normale à la surface éclairée, en filtrant le signal recueilli à travers un polariseur dont la direction de polarisation est perpendiculaire à la direction de polarisation du signal incident,
- on détermine, en balayant une gamme prédéterminée de longueur d'ondes de signal incident, un spectre de diffusion obtenu par la mesure du signal de diffusion ainsi recueilli, et
- à partir de ce spectre de diffusion, éventuellement combiné à des mesures complémentaires, on calcule une ou plusieurs grandeurs caractéristiques du comportement optique de la surface éclairée dans la gamme de longueur d'ondes prédéterminée.

Ce procédé repose essentiellement sur le fait que la ou les grandeurs caractéristiques calculées sont obtenues en tenant compte de l'énergie de diffusion émise par la surface éclairée.

Cette énergie de diffusion est en effet déterminante dans la mesure où elle rend compte des phénomènes d'absorption, de fluorescence, de réflexion et de diffraction qui se produisent dans tout milieu semi-transparent, notamment dans l'épiderme et dans le derme de la peau.

Jusqu'alors, les méthodes de mesures connues se bornaient soit à la caractérisation du comportement de la surface de la peau, indépendamment de sa pigmentation, soit à la mesure globale de la réflexion diffuse.

Afin d'accroître la pénétration du rayon lumineux dans l'épiderme et le derme, on choisit de préférence l'orientation de la polarisation du signal lumineux incident parallèle au plan d'incidence.

Selon un premier mode de mise en oeuvre de l'invention, on effectue les mesures complémentaires suivantes :

- on récupère un second signal lumineux spéculaire issu de la zone éclairée selon une direction symétrique à la direction d'incidence par rapport à la normale à la surface éclairée, en filtrant le signal recueilli à travers un polariseur dont la direction de polarisation est parallèle à la direction de polarisation du signal incident,
- on détermine, en balayant une gamme prédéterminée de longueurs d'ondes du signal incident, un spectre spéculaire obtenu par la mesure du signal spéculaire,

- on détermine un spectre de pseudo-brillance en échantillonnant le spectre de diffusion et le spectre spéculaire et en calculant, pour chaque intervalle de cet échantillonnage, le rapport entre le signal spéculaire et le signal de diffusion, ce spectre de pseudo-brillance étant caractéristique du comportement optique de la surface éclairée dans la gamme de longueurs d'ondes prédéterminée.

En particulier, on peut mettre en oeuvre ce procédé pour caractériser l'efficacité de protection d'un produit solaire.

A cet effet, on exécute le procédé décrit ci-dessus une première fois sur une zone de peau nue et l'on réitère ce procédé sur la même zone de peau après avoir recouvert cette dernière d'une pellicule du produit solaire étudié, puis on compare les deux spectres de pseudo-brillance ainsi obtenus pour en déduire, pour chaque intervalle i de l'échantillonnage, une variation de pseudo-brillance $DB_i$, et l'on calcule la grandeur suivante :

$$I = \Sigma_i \; \alpha_i \; DB_i \; K_i$$

cette valeur I étant caractéristique de l'efficacité de protection du produit,
$\alpha_i$ étant le coefficient d'efficacité érythémale dans l'intervalle considéré,
$K_i$ étant la pondération de simulation du système d'irradiation utilisé.

Les paramètres $\alpha_i$ et $K_i$ sont bien connus de l'homme du métier et sont disponibles dans la littérature spécialisée. A titre d'exemple, on peut se reporter par exemple à l'article "A comparison of in vivo and in vitro testing of sunscreens formula" dans Photochem et Photobiol, 1978, vol. 29 pp 559-566 (R. Sayres) ou au fascicule "SPF test methods", janvier 1994 du COLIPA.

L'indice ainsi obtenu peut être comparé avec l'indice de protection qui est affecté aux produits solaires actuellement commercialisés.

Selon un deuxième mode de mise en oeuvre du procédé selon l'invention, on effectue le calcul suivant :

$$ERD = \Sigma_i \; \alpha_i \; ED_i \; K_i$$

où $ED_i$ est le signal mesuré dans le spectre de diffusion pour un intervalle i de l'échantillonnage, $\alpha_i$ et $K_i$ sont respectivement les coefficients d'efficacité érythémale et les pondérations du système d'irradiation simulé.

La grandeur ERD ainsi calculée caractérise l'aptitude de la peau à résister à une irradiation dans la gamme de longueurs d'ondes considérée, en étant inversement proportionnelle à sa résistance.

L'intérêt de ce second mode de mise en oeuvre réside dans le fait qu'il procure, d'une manière quasi-instantanée, une indication de l'aptitude de la peau à résister à une exposition au soleil, ce qui requiert entre 24 et 28 heures d'expérience si l'on recourt à la méthode traditionnelle de détermination de la dose érythémale minimale.

En outre, ce procédé présente l'avantage de ne pas soumettre la peau du modèle à une irradiation qui, à la longue, pourrait entraîner des séquelles irréversibles.

La présente invention a également pour objet, selon un autre de ses aspects, un appareil pour déterminer des grandeurs caractéristiques du comportement d'une surface, notamment de la peau humaine, soumise à un rayonnement lumineux.

Cet appareil est caractérisé par le fait qu'il comporte une tête d'application contre une surface, une source lumineuse apte à envoyer un faisceau lumineux sur une zone de la surface, un polariseur placé entre la source lumineuse et la zone éclairée de la surface, le faisceau lumineux polarisé formant, avec la normale à la surface éclairée, un angle compris entre 10° et 50°, et de préférence voisin de 35°, un capteur de signal lumineux orienté pour recueillir le signal lumineux issu de la zone éclairée selon la normale à la surface éclairée, un polariseur dont la direction de polarisation est perpendiculaire à la direction de polarisation du signal incident et placé entre la surface éclairée et ledit capteur de signal lumineux, des moyens pour enregistrer, en balayant une gamme prédéterminée de longueurs d'ondes du signal incident, un spectre de diffusion obtenu par la mesure du signal lumineux recueilli par le capteur, et des moyens de calcul pour calculer à partir dudit spectre de diffusion, une ou plusieurs grandeurs caractéristiques du comportement optique de la surface éclairée dans la gamme prédéterminée de longueurs d'ondes considérée.

Dans un mode de réalisation préféré de l'invention, le premier polariseur placé entre la source lumineuse et la surface éclairée est orienté de manière à ce que la direction de polarisation du faisceau incident soit parallèle au plan d'incidence.

Dans un mode de réalisation particulier de l'invention, la source lumineuse est reliée à la tête d'application de l'appareil par l'intermédiaire d'une fibre optique, et le capteur de signal lumineux est également relié à la tête d'application de l'appareil par une fibre optique, la tête d'application de l'appareil supportant le premier et le deuxième polariseurs.

Dans un mode de réalisation préféré de l'invention, la tête d'application comporte des moyens de repérage qui permettent de l'appliquer sur une surface de peau en conservant toujours un même plan d'incidence.

Selon une variante préférée de ce mode de réalisation, l'appareil comporte à cet effet un masque applicable directement sur la peau, ce masque comportant un élément de masquage muni d'un orifice par exemple carré, tandis que la tête d'application comporte une partie de même forme apte à coopérer avec le masque lors de l'application de l'appareil sur la surface de la peau, et des supports adhésifs auxquels l'élément de masquage est relié par des moyens de fixation amovibles.

Ainsi, les supports adhésifs demeurent en permanence sur la peau, au voisinage de la surface considérée, tandis que l'élément de masquage peut être appliqué ou retiré à volonté, grâce aux moyens de fixation amovibles.

Dans ce mode de réalisation, l'appareil est toujours appliqué sur la peau par l'intermédiaire du masque avec lequel il coopère.

Lors de chaque mesure, il suffit de fixer le masque au support adhésif et d'appliquer l'appareil selon l'invention en faisant coopérer sa tête d'application avec l'orifice du masque.

L'invention a encore pour objet, selon un autre de ses aspects, un masque tel que défini à la revendication 1.

Dans le but de mieux faire comprendre l'invention, on va en décrire maintenant des modes de réalisation donnés à titre d'exemples non limitatifs en référence au dessin annexé dans lequel :

- la figure 1 est une vue en coupe axiale d'un exemple d'appareil comprenant une tête d'application,
- la figure 2 est une vue de dessous de l'appareil de la figure 1,
- la figure 3 est une vue de dessus d'un masque d'orientation selon un mode de réalisation de l'invention,
- la figure 4 est un schéma synoptique des différents éléments composant l'appareil,
- les figures 5a et 5b sont des diagrammes représentant chacun un spectre de pseudo-brillance sur une peau nue et respectivement sur une peau nue recouverte d'un produit de protection,
- la figure 5c est un diagramme représentant un spectre de variation de pseudo-brillance de ce produit,
- la figure 6 est un diagramme représentant les spectres de variation de pseudo-brillance de différents produits de protection solaire, et
- la figure 7 est un diagramme montrant la corrélation entre l'énergie de rétrodiffusion ERD au sens de l'invention et la dose érythémale minimale d'une peau.

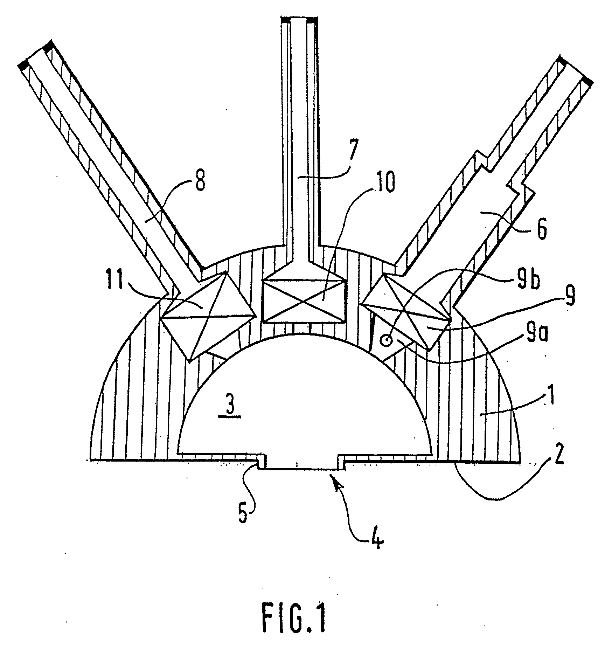

L'appareil représenté sur les figures 1 à 3 se présente sous la forme d'un boîtier semi-cylindrique 1 reposant sur son plan diamétral 2.

La cavité intérieure 3 du boîtier 1 constitue une chambre d'éclairement.

Cette chambre 3 débouche à l'extérieur du boîtier 1 par un orifice carré 4 dont les bords 5 font saillie vers l'extérieur du boîtier au-delà de son plan diamétral 2.

Dans sa partie opposée au plan 2, le boîtier 1 comporte trois canaux 6, 7 et 8 dont les directions convergent sensiblement au centre de l'orifice 4.

La direction du canal 7 est normale au plan diamétral 2, tandis que les directions des canaux 6 et 8 forment un angle d'environ 35° avec la direction du canal 7.

Chacun des canaux 6, 7, 8 débouche dans la chambre d'éclairement 3 à travers un polariseur 9, 10, 11.

L'embouchure de chaque canal est prévue pour recevoir l'extrémité d'une fibre optique.

Le canal 6 est destiné à acheminer, dans la chambre d'éclairement 3, un faisceau de lumière incident, de préférence parallèle, qui vient éclairer une zone sensiblement ovoïdale située au droit de l'orifice 4 d'une surface, non représentée, contre laquelle est appliqué l'appareil par son plan diamétral 2.

Le polariseur 9 polarise le faisceau incident de préférence parallèlement au plan d'incidence qui correspond au plan de la figure 1.

Le canal 7 recueille, grâce au polariseur 10, l'énergie lumineuse issue de la surface éclairée qui est polarisée perpendiculairement au plan d'incidence.

Ainsi, le canal 7 ne recueille que le signal lumineux diffusé par la surface éclairée et non le signal réfléchi, lequel est polarisé parallèlement au plan d'incidence comme le faisceau incident.

Le canal 8 recueille, grâce au polariseur 11, l'énergie lumineuse spéculaire polarisée dans le plan d'incidence. Cette énergie correspond à l'énergie réfléchie par la surface éclairée.

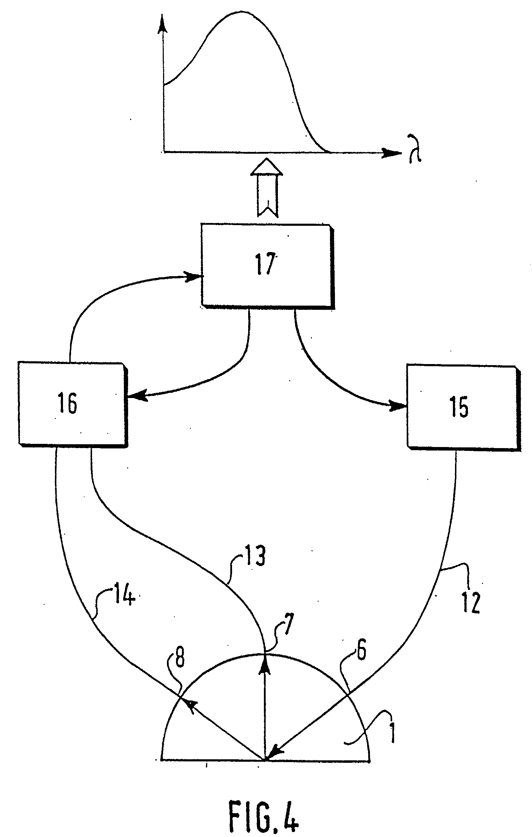

Sur la figure 4, l'appareil est raccordé à des fibres optiques 12, 13, 14 qui véhiculent les signaux lumineux incidents, diffusés et respectivement spéculaires qui traversent les canaux 6, 7 et 8.

La fibre optique 12 est reliée à une source de lumière 15, par exemple une lampe à xénon combinée avec un monochromateur, tandis que les fibres optiques 13 et 14 sont raccordées à un appareil de mesure 16, constitué par exemple par deux photomultiplicateurs et un voltmètre numérique multivoies.

Un micro-ordinateur 17 pilote la source de lumière 15 et l'appareil de mesure 16 et recueille les mesures relevées.

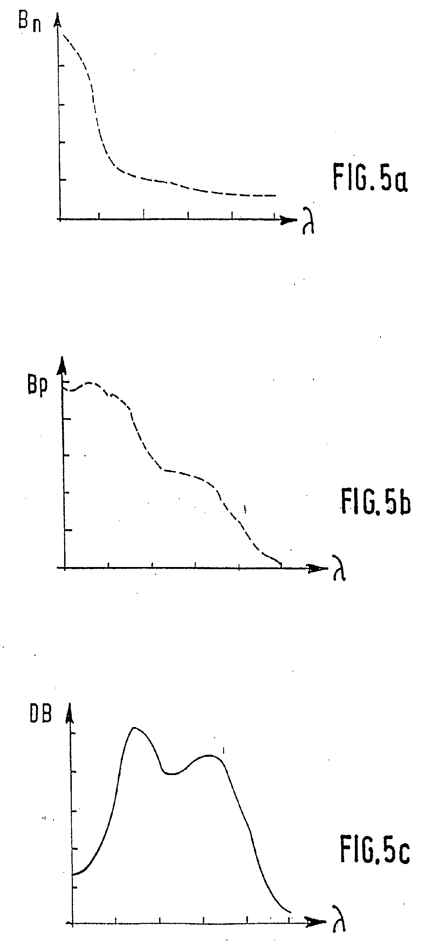

A partir de ces informations, le micro-ordinateur 17 établit un spectre de pseudo-brillance comme il va être expliqué en référence aux figures 5a, 5b et 5c.

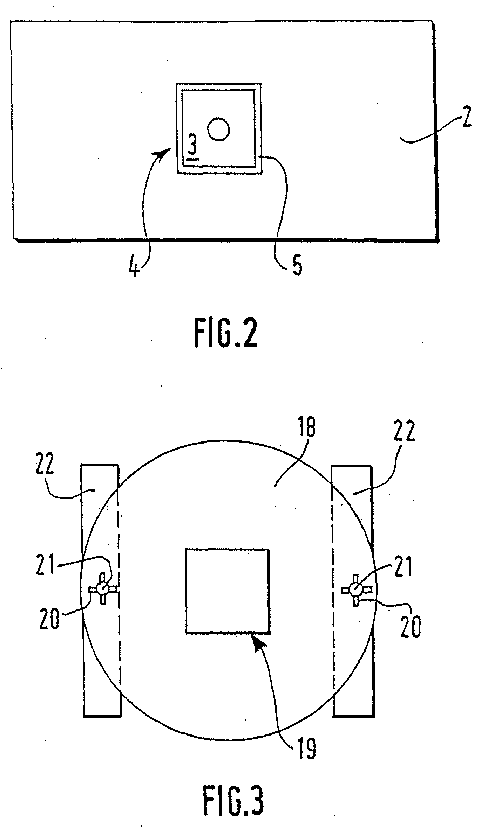

Pour son application sur la peau d'un modèle, l'appareil illustré est avantageusement combiné avec le masque qui est représenté sur la figure 3.

Ce masque comprend un disque de masquage 18 qui est muni à sa partie centrale d'un orifice 19 dont les dimensions correspondent à celles de l'orifice 4 ménagé dans le plan diamétral du boîtier 1.

Le disque de masquage 18 comporte deux passages cruciformes diamétralement opposés 20, prévus pour des têtes de rivet 21 qui sont solidaires de bandes adhésives 22.

Les bandes adhésives 22 sont destinées à être collées sur la peau du modèle, de part et d'autre de la zone de peau à étudier.

Ces bandes adhésives 22 demeurent ainsi collées pendant toute la durée de la séance de mesures tandis que le disque de masquage 18 peut être escamoté facilement.

De cette manière, on peut préparer la zone à étudier sans être gêné par le disque de masquage 18.

A chaque prise d'une mesure, on fixe le disque de masquage 18 sur les bandes adhésives 22 par l'intermédiaire des têtes de rivet 21. La zone étudiée est ainsi parfaitement délimitée par l'orifice carré 19.

On vient alors appliqué l'appareil contre le disque de masquage 18 en faisant pénétrer les bords 5 de l'orifice 4 dans l'orifice 19 du disque de masquage.

Entre deux prises de mesure, on peut retirer le disque de masquage 18 pour appliquer un traitement à la surface de peau étudiée.

Grâce à la forme carrée des orifices coopérants 4 et 19, l'appareil est toujours orienté dans une même direction lors de la prise des mesures, ce qui permet d'effectuer successivement des mesures comparatives en conservant les mêmes conditions d'éclairement de la surface de la peau.

En effet, compte tenu du relief cutané, une variation d'orientation du plan d'incidence pourrait avoir pour conséquence de fausser les mesures et d'empêcher toute comparaison significative.

Bien entendu, la forme carrée des orifices 4 et 19 peut être remplacée par toute forme non circulaire qui permet de retrouver l'orientation préférentielle du plan d'incidence.

En référence aux figures 5a, 5b et 5c, on va maintenant expliquer comment le micro-ordinateur 17 établit un spectre de pseudo-brillance.

A partir des signaux issus des canaux 7 et 8, le micro-ordinateur établit le spectre de diffusion et respectivement le spectre spéculaire de la surface étudiée, en balayant les longueurs d'ondes du faisceau lumineux incident dans une gamme de longueur d'onde prédéterminée.

Dans le cas présent, le monochromateur de la source lumineuse 15 est commandé par le micro-ordinateur 17 comme illustré à la figure 4, pour balayer par exemple une gamme de longueurs d'ondes allant de 275 à 400 nm.

Chaque spectre de diffusion spéculaire est ensuite échantillonné en petits intervalles, par exemple de 5 nm.

Pour chaque intervalle, on calcule le rapport entre l'énergie spéculaire recueillie dans le canal 8 et l'énergie de diffusion recueillie dans le canal 7.

Ce rapport définit la pseudo-brillance.

Un tel spectre de pseudo-brillance Bn est représenté sur la figure 5a, qui correspond à une mesure effectuée sur une peau nue, et sur la figure 5b qui correspond à un spectre de pseudo-brillance Bp recueilli sur la même peau, 15 mn après application d'un produit solaire à raison de 1 mg/cm2.

A partir de ces deux spectres, on peut établir, conformément à l'invention, le spectre de variation de pseudo-brillance DB qui est représenté sur la figure 5c. Ce spectre caractérise le comportement du produit à la lumière.

Ce spectre est obtenu en calculant, pour chaque intervalle i :

$$DBi = \frac{Bp(i) - Bn(i)}{Bn\ (i)}$$

Où Bp(i) est la valeur de la pseudo-brillance dans l'intervalle i sur la peau nue et Bn(i) est la valeur de la pseudo-brillance sur la peau recouverte du produit dans l'intervalle de longueur d'onde i.

Ce spectre de variation de pseudo-brillance permet de caractériser par exemple l'efficacité de protection du produit solaire considéré.

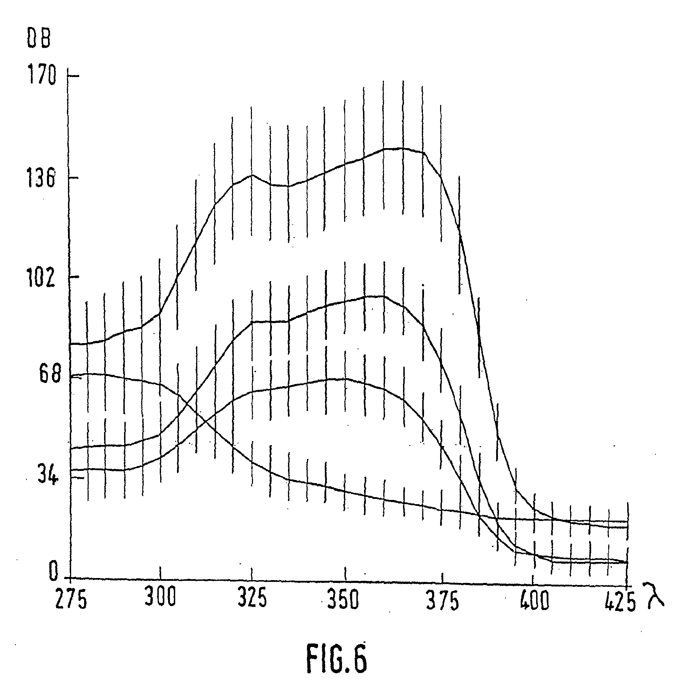

La figure 6 représente les courbes de variation de pseudo-brillance en fonction de la longueur d'onde de quatre produits solaires d'indices de protection différents.

Les quatre produits considérés A, B, C, D, ont pour composition :

| Produits | Véhicule | PARSOL 1789 (de la société Givaudan) | MEXORIL SX (de la société L'OREAL) |
|----------|----------|---------------------------------------|-------------------------------------|
| A | OUI | 0% | 0% |
| B | OUI | 0,5 % | 1% |
| C | OUI | 1% | 2% |
| D | OUI | 2% | 4% |

Le produit A est un placébo, sa courbe de variation de pseudo-brillance ne traduit que l'absorption de lumière par des éléments de la formule du placébo.

Les trois autres produits B, C, D présentent une courbe de variation de pseudo-brillance dont le maximum est atteint pour une longueur d'onde d'environ 350 nm.

Ce maximum valide la pertinence de la courbe de variation de pseudo-brillance définie selon l'invention, car on sait par ailleurs que le maximum du spectre d'absorption des trois produits testés se situe entre 350 et 355 nm.

A partir des courbes de variations de pseudo-brillance de la figure 6, on peut calculer pour chaque produit testé B, C, D, un indice caractéristique de l'efficacité protectrice du produit selon la formule suivante :

$$I = \Sigma_i \ \alpha_i \ DB_i \ K_i$$

où $\alpha_i$ est le coefficient d'efficacité érythémale à la longueur d'onde de l'intervalle considéré, et

$K_i$ est la pondération du système d'irradiation simulé.

Deux sommes sont calculées séparément. L'une pour les longueurs d'ondes couvrant le domaine des UVA, c'est-à-dire entre 320 et 400 nm, l'autre pour les longueurs d'ondes correspondant aux UVB, c'est-à-dire de 280 à 320 nm.

Une troisième somme peut être calculée sur l'ensemble du spectre, c'est-à-dire entre 280 et 400 nm.

Les indices d'efficacité de protection obtenus sont résumés dans le tableau suivant :

|  | B | C | D |
|-----|-----|-----|-----|
| UVA | 60 | 87 | 131 |
| UVB | 120 | 175 | 217 |

Ces indices sont remarquablement corellés avec les indices de protection classiques des produits B, C, D testés.

La mise en oeuvre de cette formule de détermination de l'efficacité d'un produit permet donc de calculer un indice d'efficacité de protection pour des produits nouveaux et ce, directement sur la peau d'un modèle. De cette manière, on peut déterminer différents indices d'efficacité de protection en fonction du type de peau du modèle.

On peut en particulier déterminer des échelles d'indices d'efficacité de protection adaptés par exemple aux peaux de types clair ou mat.

A partir du spectre de diffusion établi par le micro-ordinateur 17, on peut également déterminer une grandeur caractéristique de la capacité d'auto-protection d'une peau.

A cet effet, partant du spectre de diffusion ED, on calcule l'énergie de rétro-diffusion ERD par la formule suivante :

$$ERD = \Sigma_i \ \alpha_i \ ED_i \ K_i$$

Dans laquelle $\alpha_i$ et $K_i$ sont définis comme expliqué ci-dessus, et $ED_i$ est l'énergie de diffusion dans l'intervalle de longueurs d'onde i considéré.

Pour tenir compte des imperfections de la source de lumière et s'affranchir des erreurs ainsi créées qui pourraient perturber les mesures, on peut collecter également une partie du faisceau incident et corriger chaque terme $ED_i$ par un coefficient $L_i$ caractéristique de l'énergie émise par le faisceau incident dans la longueur d'onde correspondant à l'intervalle i.

Dans ce cas, l'énergie de rétro-diffusion se définit par la formule suivante :

$$ERD = \Sigma_i \; \alpha_i \; ED_i \; L_i \; K_i$$

Pour collecter une partie du faisceau incident, sur l'appareil représenté à la figure 1, on peut placer un miroir conique 9a à la sortie du polariseur 9, et dévier une partie du faisceau incident vers un orifice 9b à l'embouchure duquel des moyens de mesure et de calcul permettent de déterminer les coefficients $L_i$.

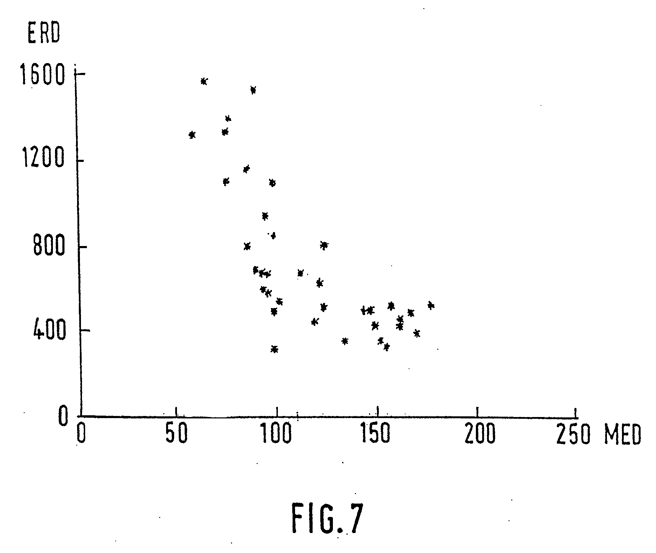

Sur la figure 7, on a reporté les valeurs calculées de l'ERD, obtenues à partir d'expérimentations faites sur le dos de plusieurs modèles dont les doses érythémales minimales varient de 60 à 182 mJ/cm$^2$.

On rappelle que la dose érythémale minimale est définie comme étant l'énergie maximale que peut supporter une peau avant de déclencher un érythème solaire.

Cette dose d'érythémale minimale est mesurée par une méthode standard qui consiste à irradier une peau et à observer, après 24 heures, la réaction de cette peau.

Sur la figure 7, il apparaît clairement que l'énergie de rétro-diffusion ERD est inversement proportionnelle à la dose érythémale minimale MED.

Ainsi, la méthode de détermination de l'énergie de rétro-diffusion ERD permet d'apprécier, a priori, la capacité d'autoprotection d'une peau et ce, d'une manière quasi instantanée, sans qu'il soit nécessaire d'irradier cette peau et d'attendre environ 24 heures.

Il est bien entendu que les modes de mise en oeuvre qui viennent d'être décrits ne présentent aucun caractère limitatif et qu'ils pourront recevoir toutes modifications désirables sans sortir pour cela du cadre de l'invention.

Le procédé et l'appareil peuvent être utilisés pour caractériser le comportement à la lumière de produits cosmétiques appliqués sur la peau, notamment des produits de maquillage.

**Revendications**

1. Masque applicable directement sur la peau, comportant un élément de masquage (18) muni d'un orifice (19) de forme asymétrique, et des supports adhésifs (22) auxquels l'élément de masquage est relié par des moyens de fixation amovibles (21).

2. Masque selon la revendication 1, **caractérisé par le fait que** l'orifice (19) est de forme carrée.

3. Ensemble comportant un masque tel que défini dans l'une des revendications précédentes, et un appareil pour déterminer des grandeurs caractéristiques du comportement de la peau soumise à un rayonnement lumineux, cet appareil comportant une tête d'application (1), **caractérisé par le fait que** la tête d'application (1) comporte une partie (4, 5) de même forme que l'orifice (19).

4. Ensemble selon la revendication précédente, **caractérisé par le fait que** la tête d'application (1) comporte des moyens de repérage (4, 5) qui permettent de l'appliquer sur une surface de peau en conservant toujours un même plan d'incidence.

5. Ensemble selon l'une des revendications 3 et 4, **caractérisé par le fait que** l'appareil comporte une source lumineuse (15) apte à envoyer un faisceau lumineux sur une zone de la surface, un polariseur (9) placé entre la source lumineuse (15) et la zone éclairée de la peau, le faisceau lumineux polarisé formant, avec la normale à la surface éclairée, un angle compris entre 10° et 50°, et de préférence voisin de 35°, un capteur (16) de signal lumineux orienté pour recueillir le signal lumineux issu de la zone éclairée selon la normale à la surface éclairée, et un polariseur (10) dont la direction de polarisation est perpendiculaire à la direction de polarisation du signal incident et placé entre la surface éclairée et ledit capteur de signal lumineux.

FIG.1

FIG.2

FIG.3

FIG. 4

FIG.5a

FIG.5b

FIG.5c

FIG.6

FIG.7